# EUROPEAN PATENT APPLICATION

(11) **EP 2 730 574 A1**
(43) Date of publication of application: **14.05.2014**
(21) Application number: 12191830.4
(22) Date of filing: 08.11.2012
(51) Int. Cl.: C07D 471/04, G01N 33/533, G01N 33/58, C12Q 1/68, G01N 21/64, G01N 33/68

(54) **Phosphorescent dye and phosphorescence immunoassay by peptide displacement**

(71) Applicant: Universität Leipzig, 04109 Leipzig (DE)
(72) Inventor: Züchner, Thole, 04275 Leipzig (DE); Kreisig, Thomas, 04179 Leipzig (DE); Nürnberger, Constance, 04105 Leipzig (DE); Hoffmann, Ralf, 04463 Grosspösna (DE); Schumer, Frank, 4056 Basel (CH)
(74) Representative: Junghans, Claas

(57) **Abstract**

The present invention relates to a method for preparation of a 2-chloro-1,10-phenanthrolinamine, comprising the steps:
i) conversion of a 2-chloro-1,10-phenanthroline to a 2-chloro-nitro-1,10-phenanthroline characterized by a formula 2,
j) hydrogenation of said 2-chloro-nitro-1,10-phenanthroline characterized by formula 5 to a 2-chloro-1,10-phenanthrolinamine characterized by formula 3.

The present invention relates further to a method for preparation of a 2-[2-[bis(carboxymethyl)amino]ethyl-[2-[carboxymethyl-[2-[4-[2-(isothiocyanato-1,10-phenanthrolin-2-yl)ethynyl]anilino]-2-oxo-ethyl]amino]ethyl]amino]acetic acid characterized by formula 7 and a method for quantifying a biomolecule in a sample.

## Description

The present invention relates to a method for preparation of an amino-reactive phosphorescent dye of formula 7. The present invention further relates to a method for quantifying a biomolecule by a luminescent immunoassay.

Immunoassays are one of the most selective analytical methods because of the high specificity of the antibody-antigen binding with up to nanomolar dissociation constants. Frequently, these assays are used for detection of proteins in complex matrices such as serum, urine or saliva. Immunoassay can be divided into heterogeneous assays and homogeneous assays.

Examples for heterogeneous assays are ELISA (Enzyme-linked immunosorbent assay) or DELFIA (Dissociation-Enhanced Lanthanide Fluorescent Immunoassay), wherein an antigen is captured by a first antibody immobilised at a solid support and visualised by a second antibody coupled to an enzyme catalyzing the conversion of a substrate to a chromophore. Heterogeneous assays are versatile and achieve a sufficient sensitivity by a signal amplification of the enzyme reaction. The disadvantage of these assays is the required great effort due to several incubation and washing steps and the long assay times.

Homogeneous assay play a crucial role for diagnostic purposes due to their shorter assay times and simpler procedures. Assay times for previously published homogenous immunoassays vary between 5 min and several hours. In homogeneous assays the reaction takes place in one solution enabling a fast und simple immunodiagnostic, because no washing or incubation steps are required. Due to the lacking separation of bound and unbound species, the detection is performed differently to heterogeneous assays. In homogeneous assays, a labelled antigen competes with the target analyte for the binding to the corresponding antibody. The unbound labelled antigen ultimately is quantified. In homogeneous FRET (Förster resonance energy transfer) -assays antigen and corresponding antibody are labelled with a donor and an acceptor dye, respectively, forming a FRET-pair, by which the spatial distance and therefore the binding state of antigen and antibody can be determined. Compared to heterogeneous assays, homogeneous assays usually show somewhat reduced sensitivities and narrower dynamic ranges. The use of conventional fluorescence dyes in homogeneous assays can be problematic because of the very high background fluorescence of sample components and microtiter plates.

Based on this background, the objective of the present invention is to provide an improved luminescent dye for homogeneous assays, especially one suitable for simple attachment to proteins. Another objective is to provide a luminescence-based homogenous immunoassay that delivers a wider dynamic range, a higher compatibility with biological matrices and an even shorter assay time.

According to one aspect of the invention, a method for preparation of a 2-chloro-1,10-phenanthrolinamine is provided, comprising the steps:
a) conversion of a 2-chloro-1,10-phenanthroline 1 (CAS-Nr. 7089-68-1) 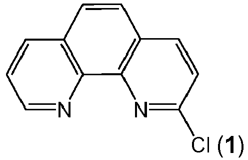 to a 2-chloro-nitropheanthroline characterized by formula 2 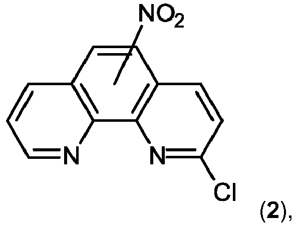
b) hydrogenation of the 2-chloro-nitro-1,10-phenanthroline characterized by formula 2 to a 2-chloro-1,10-phenanthrolinamine characterized by formula **3:**

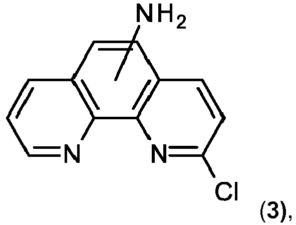

In some embodiments, the 2-chloro-1,10-phenanthrolinamine characterized by formula **3** is 2-chloro-1,10-phenanthrolin-5-amine or2-chloro-1,10-phenanthrolin-6-amine.

In one embodiment, the hydrogenation in step d) is performed in presence of a palladium catalyst.

In one embodiment, the product of step b), the 2-chloro-1,10-phenanthrolinamine characterized by formula **3,** is converted in a step c) to a protected 2-chloro-1,10-phenanthrolinamine characterized by formula **4:** 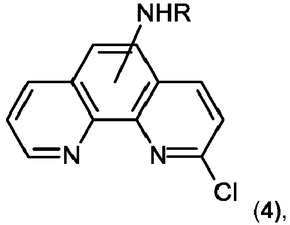 wherein R is an amine protecting group.

In one embodiment, the amine protecting group is selected from the group comprised of acetyl (Ac), phtathaloyl, acyl, carboxybenzyl (Cbz), *t*-butoxycarbonyl (Boc), allyloxycarbonyl (Aloc), benzoyl (Bz), trifluoroacetyl, benzyl, benzyloxycarbonyl and 9-fluorenylmethyloxycarbonyl (Fmoc).

In one embodiment, the product of step c), the protected 2-chloro-1,10-phenanthrolinamine characterized by formula **4,** is converted in a step d) with 4-ethynylaniline **11:** 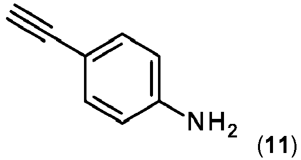 to a protected 2-[2-(4-aminophenyl)ethynyl]-1,10-phenanthrolinamine characterized by formula **5**: 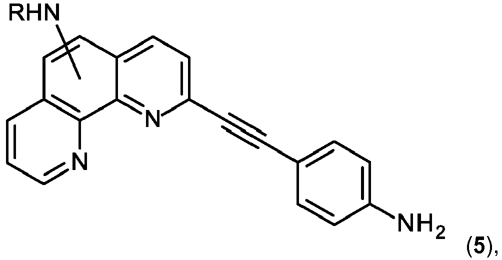 wherein R has the same meaning as above.

In one embodiment, the product of step d), the protected 2-[2-(4-aminophenyl)ethynyl]-1,10-phenanthrolinamine characterized by formula **5,** is converted in a step e) to a 2-[2-[[2-[4-[2-(amino-1,10-phenanthrolin-2-yl)ethynyl]anilino]-2-oxo-ethyl]-(carboxymethyl)amino]ethyl-[2-[bis(carboxymethyl)amino]ethyl]amino]acetic acid characterized by formula **6:**

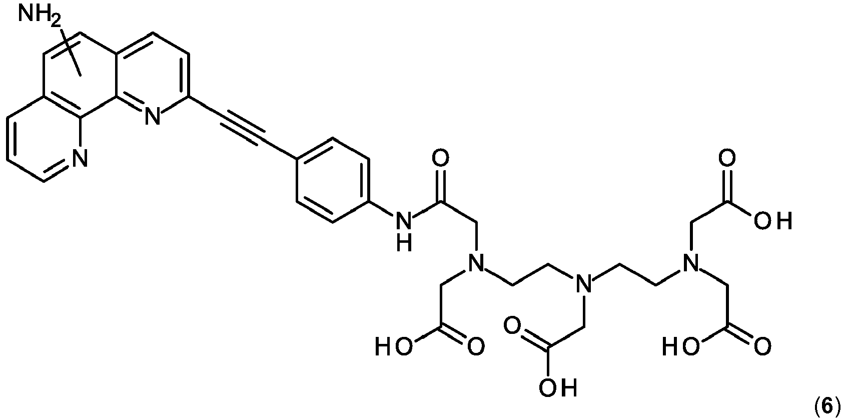

In one embodiment, the product of step e), the 2-[2-[[2-[4-[2-(amino-1,10-phenanthrolin-2-yl)ethynyl]anilino]-2-oxo-ethyl]-(carboxymethyl)amino]ethyl-[2-[bis(carboxymethyl)amino]ethyl]amino]acetic acid characterized by formula **6,** is converted in a step f) to a 2-[2-[bis(carboxymethyl)amino]ethyl-[2-[carboxymethyl-[2-[4-[2-(isothiocyanato-1,10-phenanthrolin-2-yl)ethynyl]anilino]-2-oxo-ethyl]amino]ethyl]amino]acetic acid characterized by formula **7:**

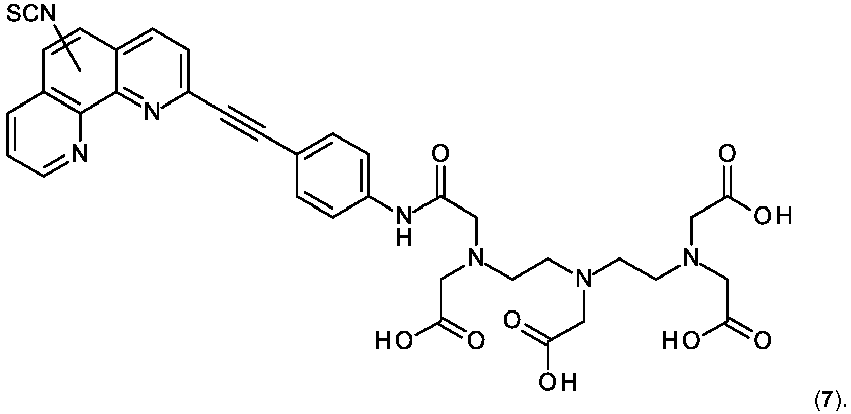

In one embodiment, the 2-[2-[bis(carboxymethyl)amino]ethyl-[2-[carboxymethyl-[2-[4-[2-(isothiocyanato-1,10-phenanthrolin-2-yl)ethynyl]anilino]-2-oxo-ethyl]amino]ethyl]amino]acetic acid characterized by formula 7 is 2-[2-[bis(carboxymethyl)amino]ethyl-[2-[carboxymethyl-[2-[4-[2-(5-isothiocyanato-1,10-phenanthrolin-2-yl)ethynyl]anilino]-2-oxoethyl]amino]ethyl]amino]acetic acid or 2-[2-[bis(carboxymethyl)amino]ethyl-[2-[carboxymethyl-[2-[4-[2-(6-isothiocyanato-1,10-phenanthrolin-2-yl)ethynyl]anilino]-2-oxoethyl]amino]ethyl]amino]acetic acid.

In one embodiment, the product of step b), the 2-chloro-1,10-phenanthrolinamine characterized by formula **3,** is converted a step g) with a compound characterized by a formula **8:** 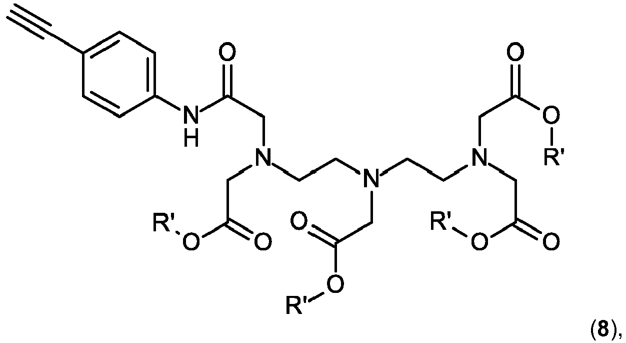 wherein R' is hydrogen, a C₁-C₄ alkyl group, benzyl (Bn) or benzhydryl, to a compound characterized by a formula 9: 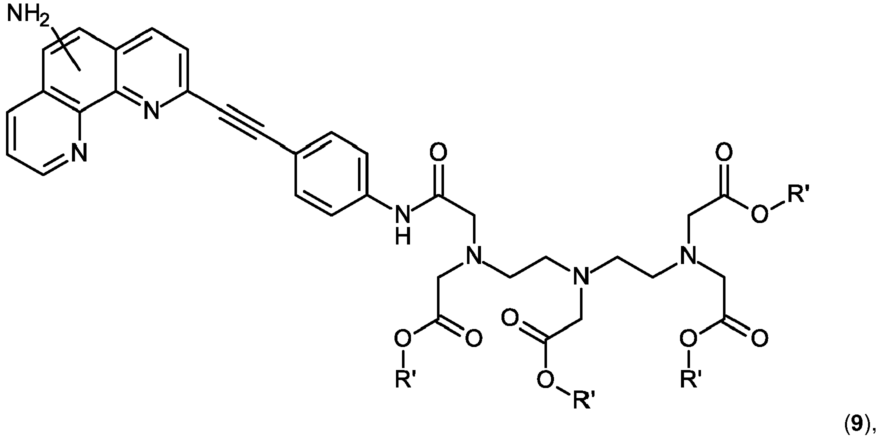 wherein R' has the same meaning as above.

A C₁-C₄ alkyl group in the context of the present invention signifies a saturated linear or branched hydrocarbon having 1, 2, 3 or 4 carbon atoms, wherein one carbon-carbon bond may be unsaturated. Non-limiting examples for a C₁-C₄ alkyl are methyl, ethyl, 1-propyl, isopropyl, prop-2-enyl, n-butyl, 2-methylpropyl, *tert*-butyl, but-3-enyl, prop-2-inyl and but-3-inyl.

In one embodiment, R' is methyl or ethyl.

In one embodiment, the compound characterized by formula 9 is converted in a step h) to a 2-[2-[bis(carboxymethyl)amino]ethyl-[2-[carboxymethyl-[2-[4-[2-(isothiocyanato-1,10-phenanthrolin-2-yl)ethynyl]anilino]-2-oxo-ethyl]amino]ethyl]amino]acetic acid characterized by formula **7.**

In one embodiment, 4-ethynylaniline is converted in a step i) with a compound characterized by formula **10:** 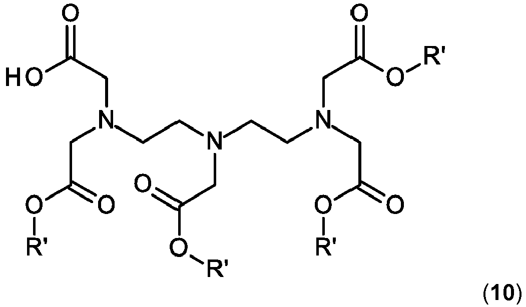 wherein R' has the same meaning as above,
to the educt of step g), a compound characterized by a formula **8.**

According to one aspect of the invention, a 2-[2-[bis(carboxymethyl)amino]ethyl-[2-[carboxymethyl-[2-[4-[2-(isothiocyanato-1,10-phenanthrolin-2-yl)ethynyl]anilino]-2-oxoethyl]amino]ethyl]amino]acetic acid characterized by formula 7 is provided.

In one embodiment, 2-[2-[bis(carboxymethyl)amino]ethyl-[2-[carboxymethyl-[2-[4-[2-(5-isothiocyanato-1,10-phenanthrolin-2-yl)ethynyl]anilino]-2-oxo-ethyl]amino]ethyl]amino]acetic acid and 2-[2-[bis(carboxymethyl)amino]ethyl-[2-[carboxymethyl-[2-[4-[2-(6-isothiocyanato-1,10-phenanthrolin-2-yl)ethynyl]anilino]-2-oxo-ethyl]amino]ethyl]amino]acetic acid are provided.

According to another aspect of the invention, a method for quantifying a biomolecule in a sample is provided, comprising the steps of
- contacting a peptide with the biomolecule, wherein the peptide is labelled with a luminescent first FRET partner, and the peptide is bound by a specifically reactive ligand with a lower affinity than the biomolecule, and
- contacting the specifically reactive ligand with the peptide and the biomolecule, wherein the specifically reactive ligand is labelled with a second FRET-partner, and the first and the second FRET partner are able to interact in such way that the luminescent signal of first FRET partner is changed with spatial approximation of the first FRET partner and the second FRET partner, and
- quantifying the amount of the biomolecule by measurement of the luminescence of the first FRET partner,
wherein the luminescent FRET partner I is a phosphorescent dye.

A first FRET partner in the sense of the invention refers to a molecule that is able to get excited by electromagnetic radiation, to emit electromagnetic radiation after excitation or to transfer energy after excitation to another molecule for example through nonradiative dipole-dipole coupling. Such first FRET partner may also be signified as donor chromophore.

A second FRET partner in the sense of the invention refers to a molecule that is able to quench the luminescence of the first FRET-Partner by energy transfer form the first FRET partner in the electronically excited state to the second FRET partner, whereby the efficiency of the energy transfer and thus the quenching rate is distance dependent. Such second FRET partner may also be signified as acceptor chromophore.

A ligand according to any aspect or embodiment of the invention may be any molecule that binds to the biomolecule with high affinity and specificity. Such ligand may be an antibody, an antibody fragment, an antibody-like molecule, an oligopeptide or a nucleic acid aptamer molecule of 10 to 75 nucleotides in length, any of which binds to the biomolecule.

High affinity in the sense of the invention refers to the dissociation constant of the binding of the ligand to the biomolecule, wherein the dissociation constant is 10⁻⁷, 10⁻⁸ or 10⁻⁹ mol/l or less, and wherein the ligand does not bind to control proteins with unrelated structural features. Control proteins are, by the way of non limiting example, plasma proteins such as albumins, globulins, lipoproteins, fibrinogens, prothrombin, acute phase proteins, tumour markers such as CEA, CA19-9 or AFP and transferrin.

High specificity in the sense of the invention refers to the ratio of properly detected biomolecule and the sum of all detected polypeptides, wherein the ratio is 80 %, 85 %, 90 %, 95 %, 99 % or 99.9 %.

An antibody fragment may be a Fab fragment, which is the antigen-binding fragment of an antibody, or a single-chain variable fragment, which is a fusion protein of the variable region of heavy and the light chain of an antibody connected by a peptide linker. An antibody-like molecule may be a repeat protein, such as a designed ankyrin repeat protein (Molecular Partners, Zürich).

Suitable ligands according to the above aspect of the invention may also be developed by evolutive methods such as phage display, ribosome display or SELEX, wherein polypeptide or oligonucleotides are selected due to their binding affinity to a target of interest. Additionally, the binding affinity of an identified ligand may be improved by cycles of evolution of the amino acid sequence or nucleotide sequence, and selection of the evolved inhibitors may be effected based on the required affinity.

In one embodiment, the phosphorescent dye is EuL[H].

In one embodiment, the peptide is obtained by conversion of the unlabelled peptide with a 2-[2-[bis(carboxymethyl)amino]ethyl-[2-[carboxymethyl-[2-[4-[2-(isothiocyanato-1,10-phenanthrolin-2-yl)ethynyl]anilino]-2-oxo-ethyl]amino]ethyl]amino]acetic acid characterized by formula 7 or a compound characterized by a formula 12: 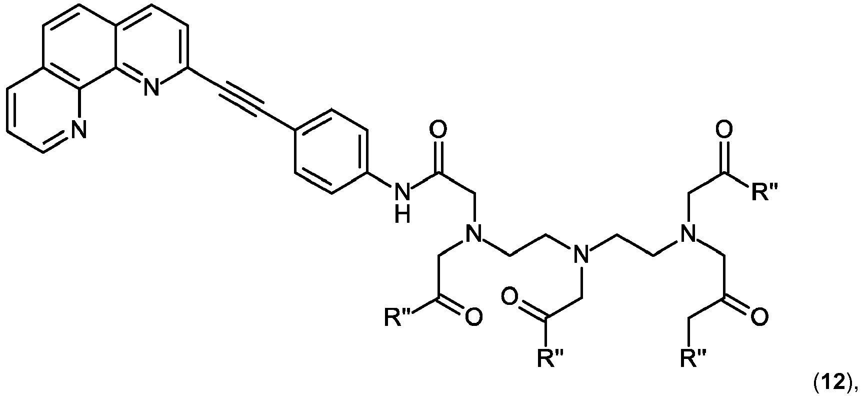 wherein each R" independently form another is selected from: 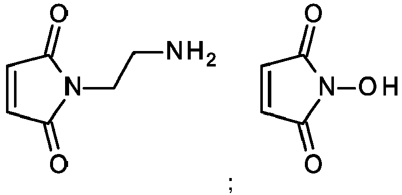 and OH.

In one embodiment, one R" is 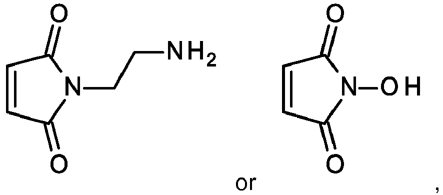 while the remaining R" are OH.

In one embodiment, the 2-[2-[bis(carboxymethyl)amino]ethyl-[2-[carboxymethyl-[2-[4-[2-(isothiocyanato-1,10-phenanthrolin-2-yl)ethynyl]anilino]-2-oxo-ethyl]amino]ethyl]amino]acetic acid characterized by formula 7 is manufactured by a method according to the above aspect or embodiments of the invention.

In one embodiment, the second FRET partner is Atto612Q or Cy5 (6-[3,3-dimethyl-2-[(1 E,3E,5E)-5-(1,3,3-trimethylindolin-2-ylidene)penta-1,3-dienyl]indol-1-ium-1-yl]hexanoic acid).

In one embodiment, the second FRET partner is not luminescent.

In one embodiment, the second FRET partner is BHQ-10 (2-[(E)-[4-[(4-hydroxy-4-oxo-butyl)-methyl-amino]phenyl]azo]-5-[(E)-(4-oxoniosulfonylphenyl)azo]benzenesulfonate).

In one embodiment, the biomolecule is the tau protein (UniProt: P10636).

UniProt. numbers refer to entries in the UniProt Knowledgebase.

In one embodiment, the specifically reactive ligand is a monoclonal antibody.

In one embodiment, the specifically reactive ligand is an immunoglobulin G.

In one embodiment, the specifically reactive ligand comprises or is a sequence selected from SEQ ID 1 and SEQ ID 2.

In one embodiment, the specifically reactive ligand is an immunoglobulin G comprising SEQ ID 1 and/or SEQ ID 2.

In one embodiment, the specifically reactive ligand comprises or is a polypeptide, wherein the polypeptide is coded by a nucleic acid with a sequence selected from SEQ ID 3 and SEQ ID 4.

In one embodiment, the peptide is VAVFR*pT*PPKSPSSAK-NH₂ or X-VAVFR*pT*PPKSPSSAK-NH₂, wherein X is mercaptopropionic acid and pT is phosphothreonine.

In one embodiment, the sample is a blood, sperm, saliva, urine or liquor sample.

According to one aspect of the invention, an ex *vivo* method for diagnosis of neurodegenerative diseases is provided, comprising a method according to the above aspect or embodiments of the invention.

In one embodiment, the neurodegenerative disease is selected from the group comprised of Alzheimer's disease, Dementia pugilistica, frontotemporal dementia, parkinsonism linked to chromosome 17 (FTDP-17), Lytico-Bodig disease (parkinson-dementia complex of Guam), tangle-predominant dementia, gangliocytoma, Meningioangiomatosis, subacute sclerosing panencephalitis lead encephalopathy, tuberous sclerosis, Hallervorden-Spatz disease, lipofuscinosis, Pick's disease and corticobasal degeneration.

According to another aspect of the invention, a polypeptide is provided, wherein the polypeptide comprises or is a sequence selected from SEQ ID 1 and SEQ ID 2.

According to another aspect of the invention, a nucleic acid is provided, wherein the nucleic acid comprises or is a sequence selected from SEQ ID 3 and SEQ ID 4

Where reference is made herein to a polypeptide characterized by a particular sequence, such reference is meant to also encompass polypeptides having an identical function to the particular sequence, and showing a sequence identity of at least 80%, 90% or 95% to the certain sequence.

Identity in the context of the present invention is a single quantitative parameter representing the result of a sequence comparison position by position. Methods of sequence comparison are known in the art; the BLAST algorithm available publicly is an example.

The invention is further characterized, without limitations, by the following examples, from which further features, advantages or embodiments can be derived. The examples do not limit but illustrate the invention.

### Description of the figures

Fig. 1 shows a reaction scheme for preparation of a phosphorescent dye of the invention.
Fig. 2 shows a schematic drawing of the method of the invention PIA (phosphorescence immunoassay).
Fig. 3 shows the chemical structures of (A) the phosphorescent donor [EuL]H and (B) the acceptor BHQ-10 (B).

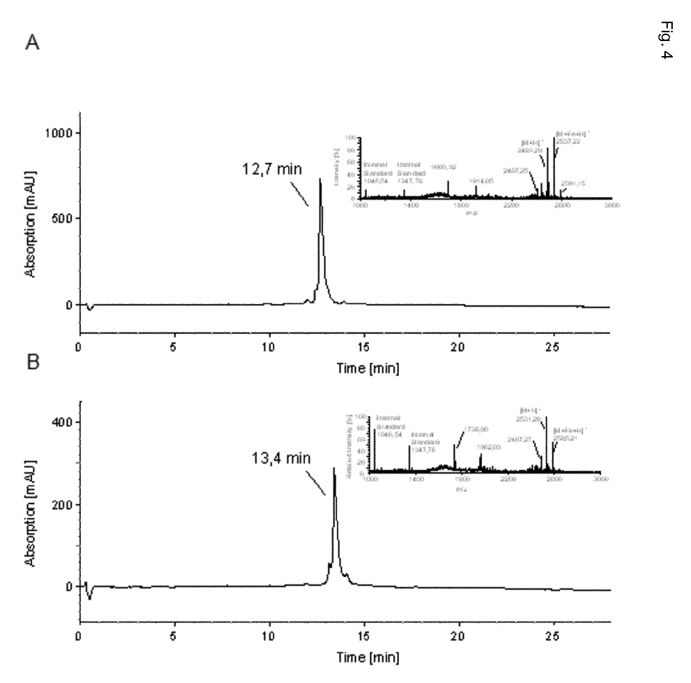

Fig. 4 shows the HPLC and MALDI-MS of synthesized donor peptides of the invention.
Fig. 5 shows the quenching efficiency of PIA compared to a fluorescence-based assay.
Fig. 6 shows the assay response of PIA using low-affinity donor peptide compared to the peptide with the native antibody recognition sequence.
Fig. 7 shows the sample compatibility of PIA to serum samples.

### Examples

### Example 1: Synthesis of the amino-reactive phosphorescent dye of the invention Nitration of 2-chloro-1,10-phenanthroline

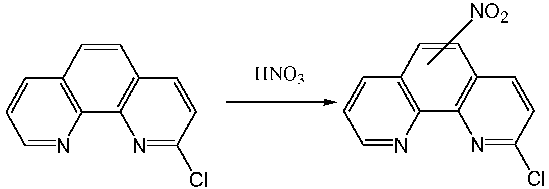

150 ml concentrated sulphuric acid was placed in a 500 ml three-necked flask and 2-Chloro-1,10-phenanthroline **1** (CAS-Nr. 7089-68-1)(30g) was added while cooled with ice and stirred. The solution was heated in an oil bath to 180 °C. At 80 °C, the solution boiled and a strong smoke emission could be observed. At 160 °C, nitric acid (65 %, 80 ml) was added dropwise while maintaining a temperature of 170 °C. The bath temperature was increased to 215 °C, and the adding was performed in such a way that the temperature was below or equal to 160 °C. After complete addition of the nitric acid, the solution was heated under reflux for 1 h to 165 to 170 °C. Following this, the reaction mixture was cooled in a ice bath, added to approximately 1 kg ice and diluted with 1 l water. A sodium hydroxide solution (50 % w/v) was added dropwise until the pH value of the reaction mixture was in a neutral/weak alkaline range. This was accompanied by formation of a slightly yellow precipitate. The precipitate was separated and washed with water. The crude product of the reaction was dried over night at 110 °C, extracted with chloroform via Soxleth extraction and reduced to a dry powder in rotary evaporator.

### Reduction of 2-chloro-5/6-nitro-1,10-phenanthroline

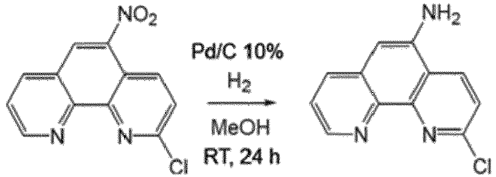

2-Chloro-5/6-nitro-1,10-phenanthroline **2** (1 eq.) was dissolved in methanol and hydrogenated under stirring in the presence of the catalyst (palladium/coal 10%, 10mol%) in a hydrogenation plant until no further hydrogen consumption could be observed. The catalyst was filtered off and the filtrate was reduced to dryness under reduced pressure (complete conversion).

### Introduction of a trifluoro acetyl group to 5/6-amino-2-chloro-1,10-phenanthroline

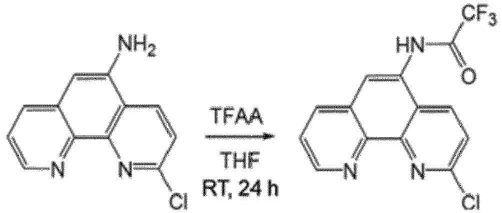

5/6-Aminochlorophenanthroline **3** (230 mg, 1 mmol) was suspended in 20 ml water-free THF and tempered to 0 °C. Then, trifluoroacetic acid was added (275 µl, 3mmol). The initially formed precipitate dissolved after 30 min. The reaction was performed until completion under stirring for 24 hours at room temperature. The reaction mixture was reduced to a dry powder in rotary evaporator. Then, 20 ml water was added the dry substance, and the pH-value of the solution was adjusted with ammonia (25%) to 8. The aqueous phase was extracted 3 times with 50 ml chloroform, respectively. The organic phase was dried over Na₂SO₄, reduced to a dry powder in a rotary evaporator. The crude product was purified with silica gel 60 (m=33 g) and chloroform/methanol (9/1; v/v) as eluent. Fractions containing pure product (examined by thin layer chromatography) were unified and reduced to a dry powder in a rotary evaporator (m=205 mg, 63% yield).

### Introduction of a Boc group to 5/6-amino-2-chloro-1,10-phenanthroline

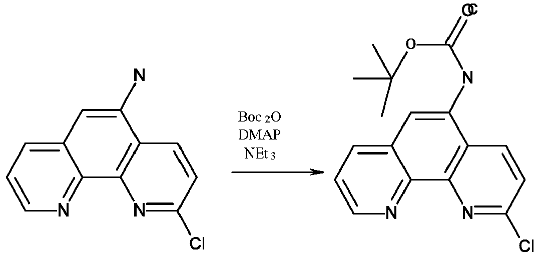

Alternatively, the amine group of 2-chloro-1,10-phenanthroline 3 may be protected with a Boc group. To do this, 5/6-amino-2-chloro-1,10-phenanthroline (1 eq.) was dissolved in dry DMF and triethylamine (5 eq.). Then, DMAP (1.1 eq.) and di-*t*-butyldicarbonate (2 eq.) were added The solution was stirred at room temperature for 20 h. Then, water was added and the aqueous phase was extracted three times with diethylether. The combined organic phases were dried over Na₂SO₄ and concentrated to dryness. The crude product was purified with silica gel (CH₂Cl₂). (63% yield).

### Coupling of TFA-protected 2-chloroaminophenanthroline and ethinylaniline

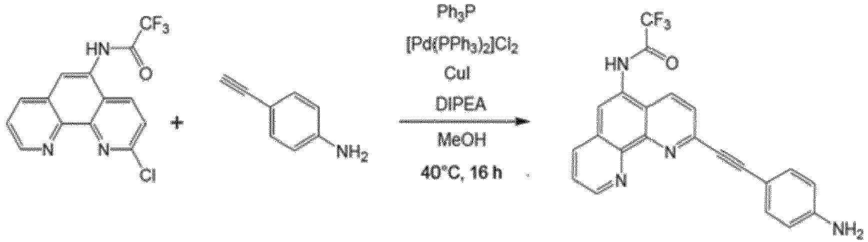

Triphenylphosphine (30 mg, 115 µmol), [Pd(PPh₃)₂]Cl₂ (20 mg, 285 µmol) and copper(I)iodide (16 mg, 84 µmol) were placed in a 100 ml three-necked flask and suspended in 30 ml methanol. The educt trifluoroacetyl-5/6-aminochlorophenanthroline **4** (205 mg, 466 µmol) was transferred into the flask with 20 ml methanol. The reaction mixture was tempered to 40 °C, and nitrogen was fed into. Ethinylaniline (200 mg, 1.7 mmol) was solved in a few millilitres methanol and added to the reaction mixture. Then, DIPEA (N,N-diisopropylethylamine, 2 ml) was added, and the reaction mixture was stirred over night. After this, mixture was extracted three times with 50 ml chloroform, respectively. The chloroform phase was washed with diluted sodium bicarbonate solution, diluted hydrochloric acid and again with sodium bicarbonate solution. Then, the chloroform phase was dried over Na₂SO₄ and purified with an aluminium oxide column and chloroform.

### Coupling of the Boc-protected 2-chloro-5/6-amino-1,10-phenanthroline and 4-ethinylaniline

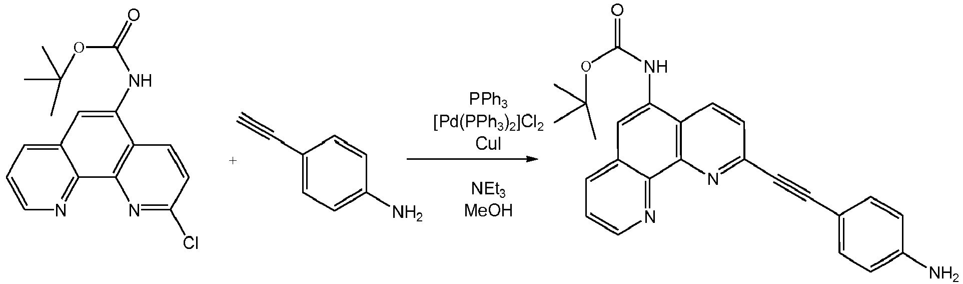

Triphenylphosphine (30 mol%), [Pd(PPh₃)₂]Cl₂ (3mol%), the protected 2-chloro-5/6-amino-1,10-phenanthroline **4** (1 eq.) and copper(I)iodide (2mol%) were placed in a three-necked flask and suspended in 30 ml methanol. The 4-ethinylaniline (1.2 eq.) was added dissolved in 20 ml methanol. The reaction mixture was refluxed for 10 h. The mixture was extracted three times with dichloromethan, respectively. The combined organic phases were washed with water and brine before being concentrated to dryness. Purification by flash-chromatography yielded compound **5** as a brown solid.

### Coupling of the Boc-protected [2-[(4'-aminophenylen)ethynylen]-1,10-phenanthrolin-5/6-yl]amine and DTPA-anhydride

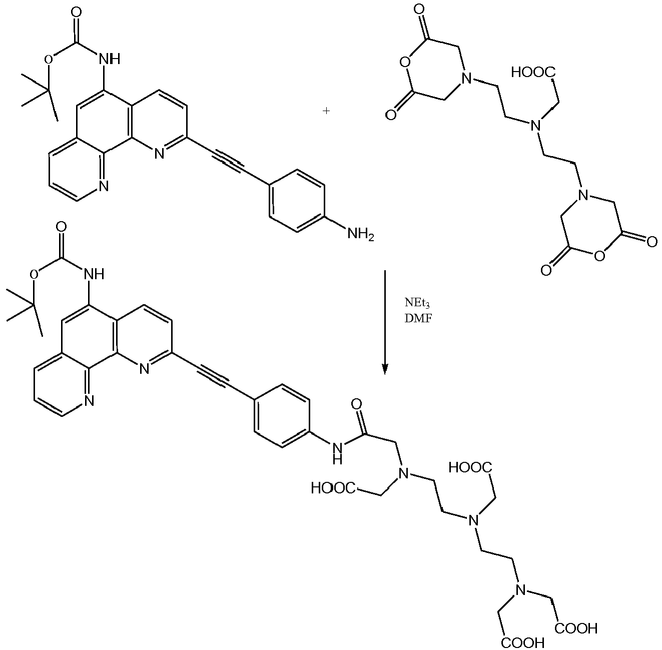

[2-[(4'-Aminophenylen)ethynylen]-1,10-phenanthrolin-5/6-yl]amine **5** (1 eq.), DTPA-dianhydride and triethylamine (5 eq.) were stirred in DMF under argon for 2 h at 80°C. The sovents were removed under reduced pressure. The residue was purified by RP-HPLC (water/MeCN).

### Coupling of ethinylaniline with a tetraprotected DTPA-

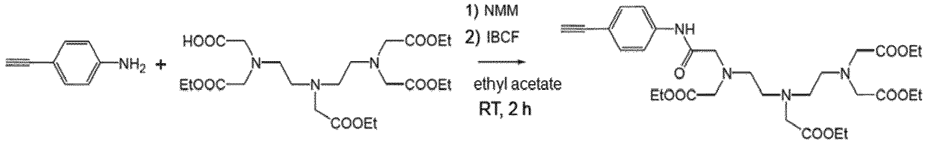

As an alternative to the approach described above and to exclude the need of protection group at the 2-chloro-5/6-amino-1,10-phenanthroline 3, ethinylaniline was converted with an tetraprotected DTPA 10, i.e. DTPATE (diethylene triamine pentaacetate tetraethyl ester). In a three-necked flask DPTATE (1 eq.) was dissolved in dry ethyl acetate. NMM (N-methyl morpholine) was added under stirring. Then, the reaction mixture was tempered to 0 °C and isobutyl chloroformate was added, whereby a colourless precipitate formed. Then, the mixture was stirred at room temperature for 2 hours. The reaction mixture was cooled to - 20 °C, and the cooled suspension was filtrated. The filtrate was cooled again and additional formed precipitate was filtrated. Then, ethinylaniline (1 eq., dissolved in ethyl acetate) and a second portion of NMM were added. The reaction mixture was stirred under a nitrogen athmosphere at room temperature over night. The reaction mixture was then reduced to dryness. The crude product was purified with a silica gel (ethyl acetate/petrol ether, 3:2).

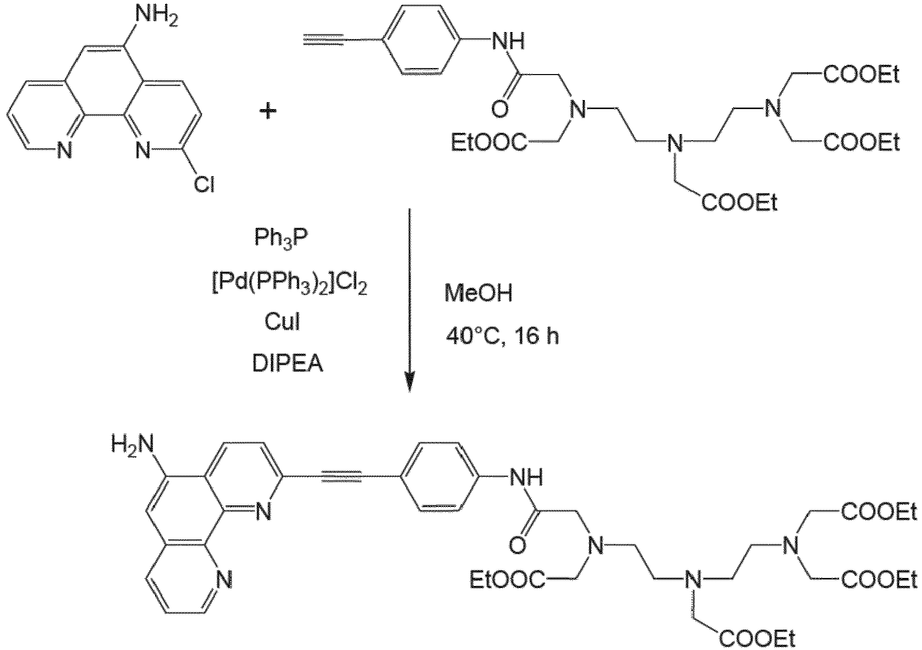

The product **8** of this reaction was converted with the unprotected 5/6-amino-2-chloro-1,10-phenanthroline **3,** similar to the coupling reaction of the protected 5/6-amino-2-chloro-1,10-phentanthroline **4** with ethinylaniline.

### Conversion of 2-[2-[[2-[4-[2-(5/6-amino-1,10-phenanthrolin-2-yl)ethynylen]anilino]-2-oxoethyl]-(carboxymethyl)amino]ethyl-2-[bis(carboxymethyl)amino]ethyl]amino]acetic acid 6 to 2-[2-[bis(carboxymethyl)aminolethyl-[2-[carboxymethyl-[2-[4-[2-(5/6-isothiocyanato-1,10-phenanthrolin-2-yl)ethynylen]anilino]-2-oxo-ethyl]amino]ethyl]amino]acetic acid 7

2-[2-[[2-[4-[2-(5/6-amino-1,10-phenanthrolin-2-yl)ethynylen]anilino]-2-oxo-ethyl]-(carboxymethyl)amino]ethyl-[2-[bis(carboxymethyl)amino]ethyl]amino]acetic acid **6** (1 eq.) was dissolved in water, slowly added to the mixture of thiophosgen (6.8 eq), sodiumhydrogen carbonate (9.9 eq.) and chloroform and stirred for 1.5 h. The phases were separated and the aqueous phases were washed with chloroform. The pH was adjusted to neutral and the product was precipitated with acetone.

### Example 2: Assay principle

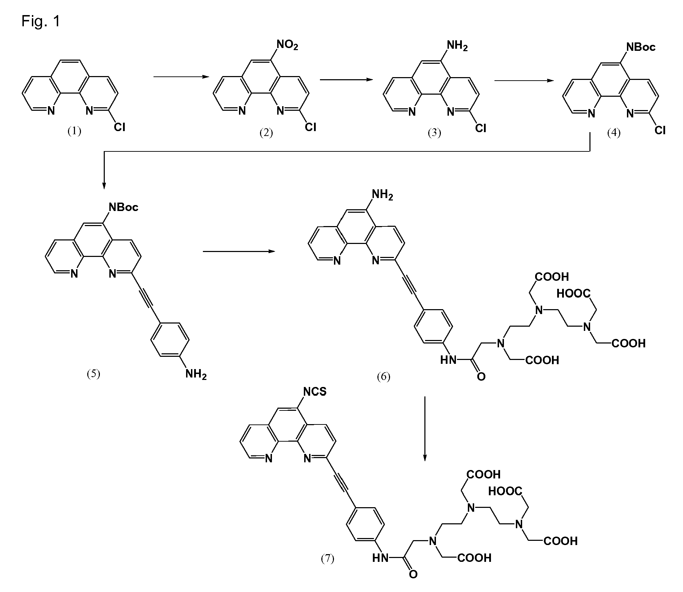

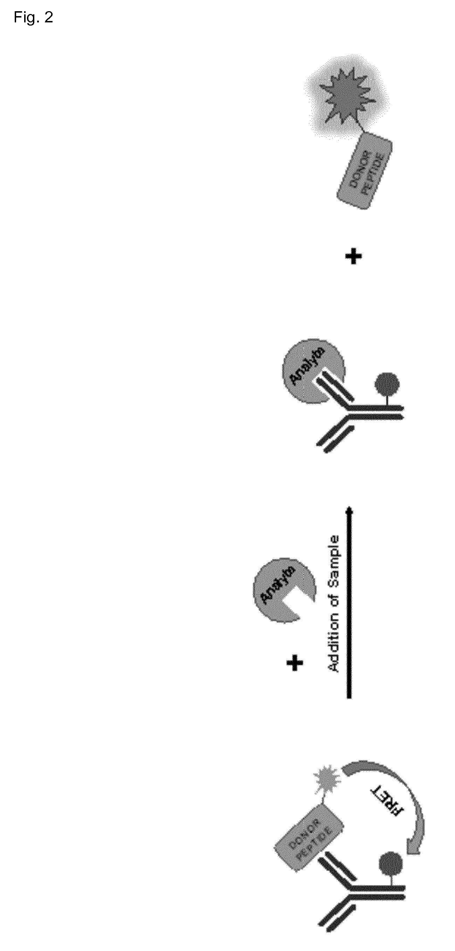

As shown in Fig. 1, the donor peptide wears the phosphorescent donor [EuL]H (Fig. 2) and is recognized by the corresponding antibody. The antibody HPT-104, an immunoglobulin G with an amino acid sequence comprising SEQ ID 1 and SEQ ID 2, is labeled with BHQ-10 (Fig. 2) as acceptor dye. The immunocomplex of both shows a low donor emission. This is due to Förster resonance energy transfer (FRET), where the energy of the excited donor molecule transfers non-radiatively to the acceptor dye. This phenomenon is strongly dependent on the distance between donor and acceptor. In the immunocomplex, the distance is short enough to obtain a sufficient FRET. When a sample containing the target analyte is added to this solution, the analyte displaces the donor peptide from the acceptor labeled antibody leading to an increase in donor emission intensity due to a much longer distance between donor and acceptor. The resulting signal depends on the concentration of the analyte in the given sample.

### Example 3: Synthesis of the donor peptides of the invention

### Peptide synthesis

All peptides were synthesized by the Fmoc/*tert*-butyl strategy on Rink amide resin using a peptide synthesis robot Syro 2000. The resin was swollen 30 min in DMF, and the terminal Fmoc group was cleaved with 40 % piperidine in DMF (v/v). Then, the amino acid coupling was performed following standard procedure. After the robotic synthesis the peptide resins were washed six times with dichloromethane and dried under vacuum.

After solid phase synthesis a small portion of the peptide resin was cleaved for one hour at room temperature with 150 µl cleavage solution (5% water, 4% thioanisole, 4% m-cresol and 2 %ethanedithiol in TFA). Then, the cleavage solution was filtrated into a 1.5 ml reaction vessel. By adding 1 ml ice-cold diethyl ether the peptide was precipitated. The suspension was centrifugated after 5 min incubation on ice. The supernatant was discarded, and the peptide pellet was washed twice with ice-cold diethyl ether, dried in an exhaust hood and solved in 200 µl 3% acetonitrile solution in water with 0.1% TFA. This solution was directly deployed for RP-HPLC analysis. For MALDI-TOF analysis, the solution was diluted 100-fold with the same solvent.

The residual amount of peptide resin was cleaved with 1 ml cleavage solution and fairly shaken. The cleavage solution was filtrated in a 1.5 ml reaction vessel. For complete cleavage the peptide resin was added to 0.5 ml cleavage solution, fairly shaken for 1.5 hours and filtrated. The unified filtrates were precipitated with ice-cold diethyl ether, centrifugated (1620 g, 5 min), and the supernatant was discarded. The peptide pellet was washed twice with ice-cold diethyl ether (8 ml) and dried in an exhaust hood over night. For chromatographic purification the crude peptide was solved in 3% acetonitrile solution in water with 0.1% TFA and centrifugated separating insoluble components.

### Phosphorylation

The N-terminus of donor peptide of the invention was coupled to mercaptopropionic acid instead of cysteine. The unprotected side chain of Thr231 (corresponding to Thr231 of the analyte peptide Tau226-240=native peptide) was globally phosphorylated with the phosphoramidite method. The peptide resin was placed in a 15 ml reaction vessel and swollen with dry DMF (2 ml). The vessel was hermetically closed, the suspension was rinsed 20 min nitrogen and the DMF was removed by a syringe. The following reaction steps were performed under nitrogen atmosphere. Each peptide resin was reacted with tetrazole (30 eq) and dibenzyl-N,N-diisopropylphosphoramidite (15 eq: diluted tenfold in dry DMF) for 90 min at room temperature. Then, the reaction solution was removed by a syringe, and the reagents were added again and mixed over night. After this, this reaction solution was removed again, and the resin was washed twice with DMF (2 ml) and oxidized for 90 min with *tert*-butyl hydroperoxide (100 eq; 5.5 mol/l in decane). The reaction solution was removed, and *tert*-butyl hydroperoxide was added again to the resin and mixed for 90 min. Then, the solution was removed, and the resin was washed four times with DMF, four times with dichloromethane and was dried. The reaction was inspected by MALDI-TOF-MS analysis. After inspection, the phosphorylated peptide was completely cleaved of the resin. The analyte peptide (Tau226-241) was phophorylated as described above. The cleaved peptides were purified with RPC (C₁₈ column ; 21%-36% acetonitrile in 30 min, 0.1 % TFA).

### Coupling of [EuL] to donor peptides

[Eul]H was solved in dry DMF (20 mg; 110 µl DMF) and mixed with 2-aminoethylmaleimide trifluoroacetate (2.5 eq; 19.1 mg in 40 µl DMF). The reaction was started under nitrogen by adding DIC (diisopropylcarboiimide). The reaction vessel was cooled in the first 30 minutes, then shaken at room temperature for 2 hours. The product was purified with RPC (reversed phase chromatography) with a C₁₈ column and an aqueous acetonitrile gradient (10%-35% acetonitrile in 30 min; 0.1% TFA).

The phosphorylated donor peptide (2 mg/ml in PBS [phosphate buffered saline]) was mixed with the maleimid-activated dye [EuL]H described above (5 eq; 0.45 mol(l) in DMF) and incubated for 2 hours at room temperature. The product of the reaction was purified with a C₁₈-column and an aqueous acetonitrile gradient (18%-36% acetonitrile in 30 min; 5 mmol/l triethylammonium formate; pH 8.5). For use of the peptides in the assay of the invention, an aqueous EuCl₃ solution was added, whereby the Eu³⁺ ions were in a 1.5fold excess to the peptides.

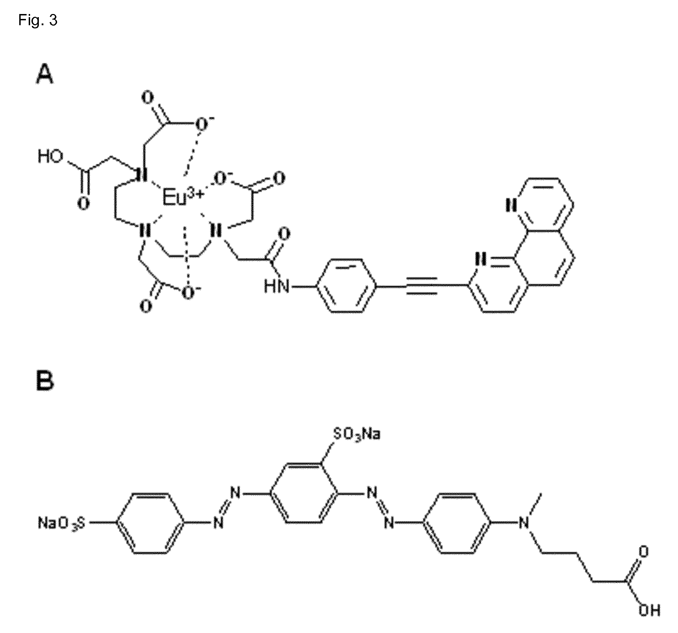

Fig. 3 shows the RP-HPLC chromatograms and the results of the MALDI-MS of [EuL]H-peptide with native antibody recognition sequence (Fig. 3A): [EuL]H-XVAVVR*pT*PPKSPSSAK-NH₂ (X - mercaptopropionic acid; pT- Phosphothreonine), Fig. 3B shows the low-affinity [EuL]H-peptide (Fig. 3B): [EuL]H-XVAVFR*pT*PPKSPSSAK-NH₂. Both RP-HPLC chromatograms indicate pure peptides with purities ≥94%. The MALDI-MS results confirm the identity of these peptides.

### Example 4: Comparison of PIA with a fluorescence immunoassay

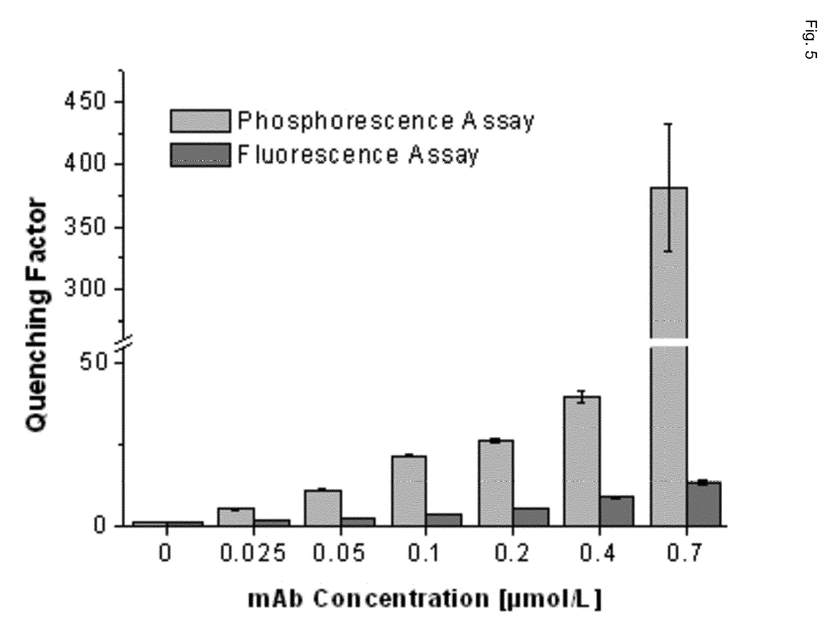

Only the donor peptide (fixed concentration) was mixed with increasing concentrations of acceptor-labeled HPT-104 antibody (Fig. 5). The antibody was labeled with BHQ-10 (2-[(E)-[4-[(4-hydroxy-4-oxo-butyl)-methyl-amino]phenyl]azo]-5-[(E)-(4-oxoniosulfonylphenyl)azo]-benzenesulfonate) as acceptor using the succinimidyl ester of BHQ-10 in access to the antibody in phosphate buffered saline. The antibody solution (2.1 g/l in PBS) was added to 20 eq acceptor dye solved in DMF (7.1 mmol/l), strongly mixed with a vortex and incubated for 2 hours at room temperature at a dark place. Then, the reaction mixture was centrifugated for 5 min at 1620 rpm and separated from unreacted dye with a HiTrap desalting column (isocratic; eluent PBS; 5 ml/min). The purified antibody fractions were unified, mixed with 0.001% sodium azide and stored at 4 °C. The affinity and specificity was confirmed by ELISA. The measured donor emission decreases due to FRET. The quenching factor is calculated by dividing the intensity of the control, where no antibody was added, by the emission of the quenched intensity. The resulting quenching curve of the phosphorescence assay was compared to a curve using the same peptide but labeled with fluorescein instead of [EuL]H (= fluorescence assay) (Fig. 5). The results demonstrate the strong quenching of using [EuL]H and phosphorescence detection compared to the fluorescence assay.

### Example 5: Comparison of the assay response of the low-affinity donor peptide of the invention with native peptide

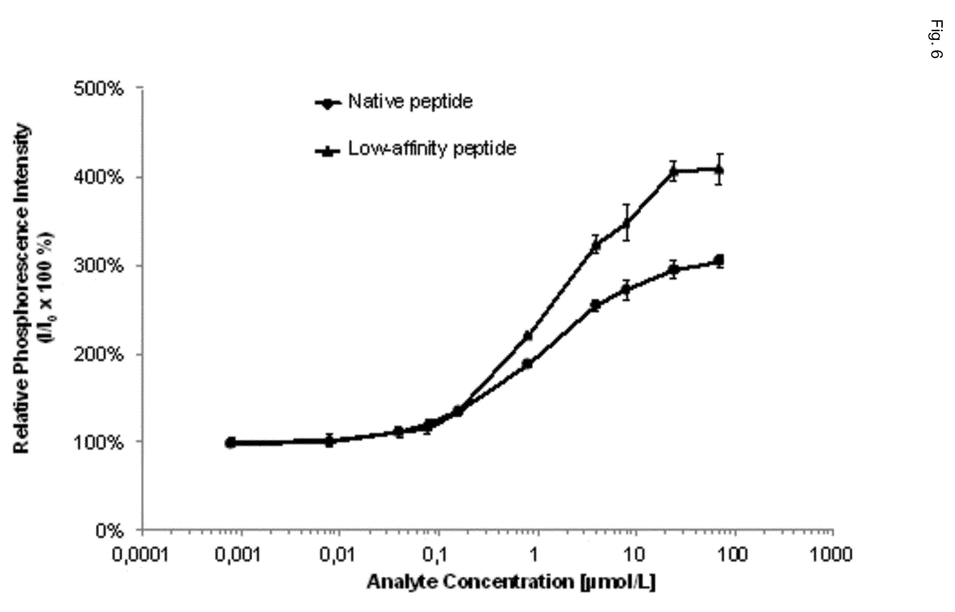

The phosphorescence assay was examined using the low-affinity [EuL]H-peptide in comparison to the native [EuL]H-peptide (Fig. 6). The analyte sample with different concentrations was spiked with a fixed concentration of donor peptide and mixed directly afterwards with the acceptor labeled antibody. The [EuL]H emission was recorded after a 90s shaking step. The assay using the low-affinity peptide produced a stronger signal increase than the native [EuL]H-peptide. The low-affinity [EuL]H-peptide was more efficiently replaced by the analyte compared to the native [EuL]H-peptide.

### Example 6: Sample compatibility of PIA to serum samples

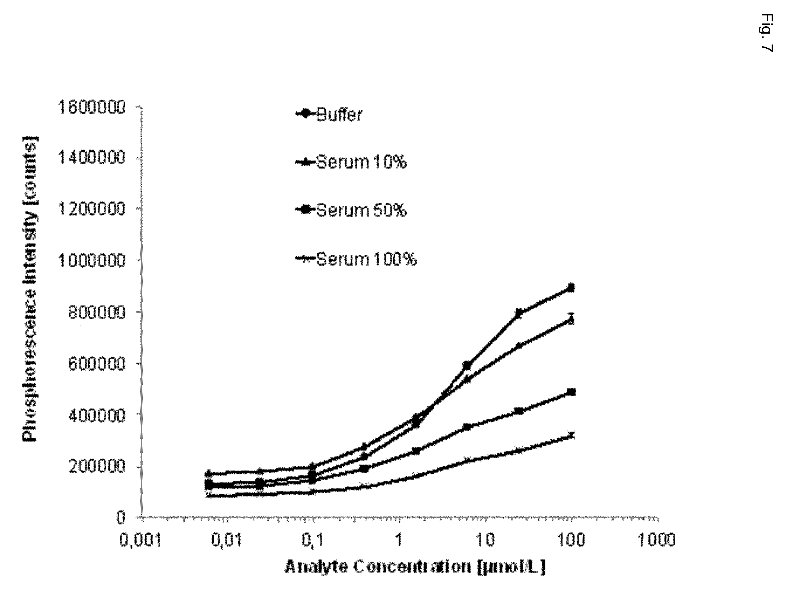

The PIA (using the low-affinity [EuL]H-peptide) was used to measure analyte samples with increasing concentrations (Fig 7). The analyte sample was diluted in different porcine serum concentrations up to 100% as well as in buffer as a control. Even in undiluted porcine serum (100%), there is a signal increase with increasing analyte concentration. For 10% serum the signal curve is almost identical with the curve in buffer, demonstrating the high compatibility of PIA to serum samples.

### Example 7: Intra- and interassay variation and recovery rates of PIA

The intraassay variation was calculated from the results of five independent replicates within one assay. The interassay variation was calculated from three separate assays. Recovery rates were determined as quotient between the analyzed concentration of three analyte samples and their known concentrations. All of these parameters demonstrate acceptable values for homogeneous assays and prove the feasibility of PIA (table 1).

**Table 1:**

| **Intraassay** | | | | **Interassay** | | | **Recovery rates** | | |
|---|---|---|---|---|---|---|---|---|---|
| Sample | Mean | STD | CV | Mean | STD | CV | Known | Found | Recovery |
| | Conc. | | [%] | Conc. | | [%] | Conc. | Conc. | [%] |
| | [µmol/L] | | | [µmol/L] | | | [µmol/L] | [µmol/L] | |
| | | | | | | | | | |
| 1 | 0,75 | 0,05 | **7,3** | 0,85 | 0,09 | **11,1** | 0,80 | 0,75 | **93,8** |
| | | | | | | | | | |
| 2 | 1,63 | 0,04 | **2,5** | 1,73 | 0,13 | **7,8** | 1,60 | 1,63 | **101,7** |
| | | | | | | | | | |
| 3 | 3,30 | 0,42 | **12,6** | 3,38 | 0,08 | **2,3** | 3,20 | 3,30 | **103,1** |

## Claims

1. A method for preparation of a 2-[2-[bis(carboxymethyl)amino]ethyl-[2-[carboxymethyl-[2-[4-[2-(isothiocyanato-1,10-phenanthrolin-2-yl)ethynyl]anilino]-2-oxoethyl]amino]ethyl]amino]acetic acid (7), wherein
a) 2-chloro-1,10-phenanthroline is converted to a 2-chloro-nitro-1,10-phenanthroline **characterized by** formula 2: 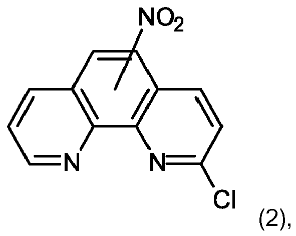
b) said 2-chloro-nitro-1,10-phenanthroline **characterized by** formula 2 is hydrogenated to a 2-chloro-1,10-phenanthrolinamine **characterized by** formula 3: 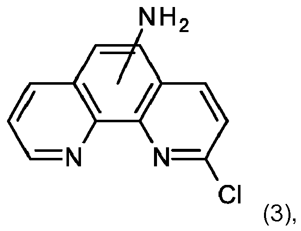
c) said 2-chloro-1,10-phenanthrolinamine **characterized by** formula 3 is converted to a protected 2-chloro-1,10-phenanthrolinamine **characterized by** formula 4: 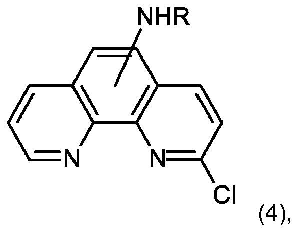 wherein R is an amine protecting group,
d) said protected 2-chloro-1,10-phenanthrolinamine **characterized by** formula 4 is converted with 4-ethynylaniline to a protected 2-[2-(4-aminophenyl)ethynyl]-1,10-phenanthrolinamine **characterized by** formula 5: 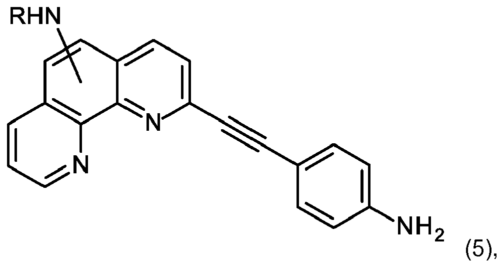 wherein R has the same meaning as above,
e) said protected 2-[2-(4-aminophenyl)ethynyl]-1,10-phenanthrolinamine **characterized by** formula 5 is converted to a 2-[2-[[2-[4-[2-(amino-1,10-phenanthrolin-2-yl)ethynyl]anilino]-2-oxo-ethyl]-(carboxymethyl)amino]ethyl-[2-[bis(carboxymethyl)amino]ethyl]amino]acetic acid **characterized by** formula 6: 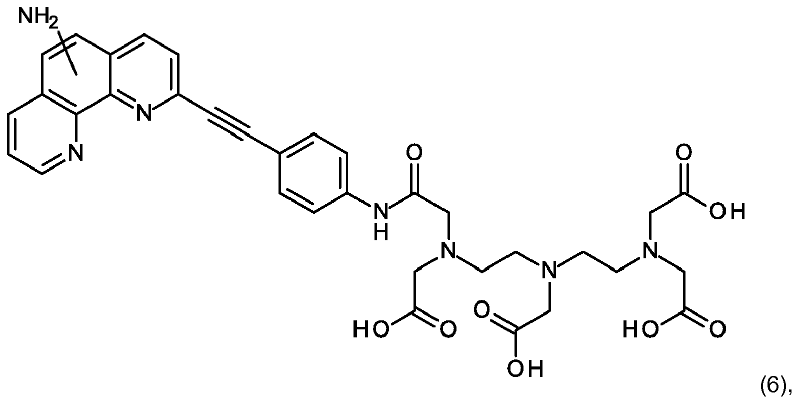 and
f) said 2-[2-[[2-[4-[2-(amino-1,10-phenanthrolin-2-yl)ethynyl]anilino]-2-oxo-ethyl]-(carboxymethyl)amino]ethyl-[2-[bis(carboxymethyl)amino]ethyl]amino]acetic acid **characterized by** formula 6 is converted to a 2-[2-[bis(carboxymethyl)amino]ethyl-[2-[carboxymethyl-[2-[4-[2-(isothiocyanato-1,10-phenanthrolin-2-yl)ethynyl]anilino]-2-oxo-ethyl]amino]ethyl]amino]acetic acid **characterized by** formula 7: 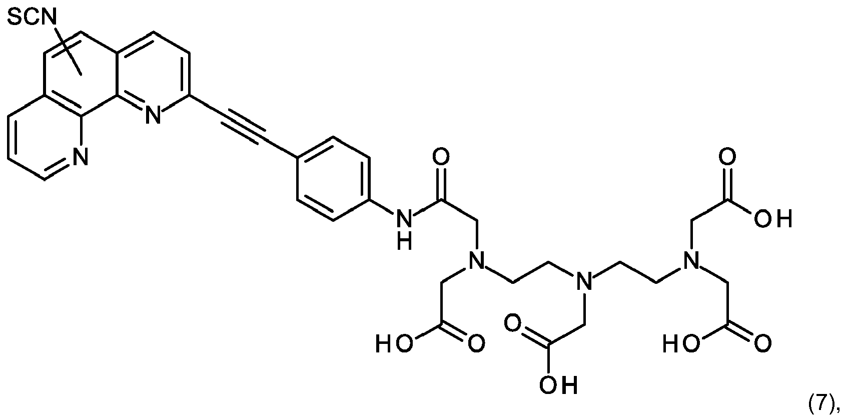 or
g) said 2-chloro-1,10-phenanthrolinamine **characterized by** formula 3 is converted with a compound **characterized by** formula 8: 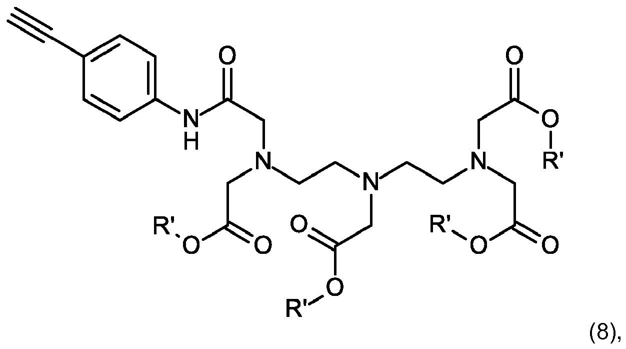 wherein R' is hydrogen or a C₁-C₄ alkyl group, benzyl (Bn) or benzhydryl, to a compound **characterized by** formula 9: 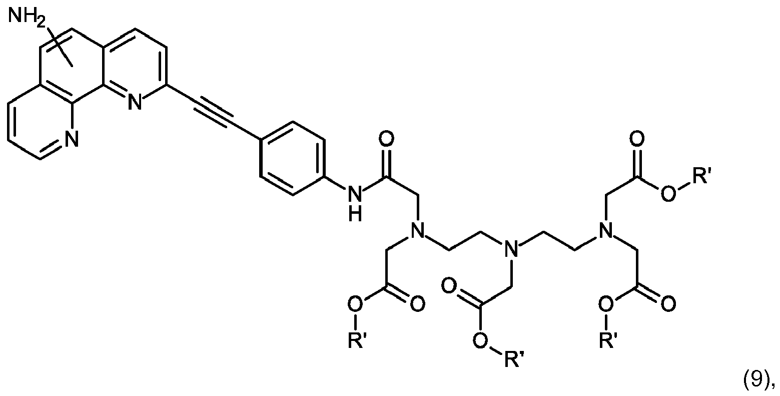 wherein R' has the same meaning as above, and h) said compound **characterized by** formula 9 is converted to a 2-[2-[bis(carboxymethyl)amino]ethyl-[2-[carboxymethyl-[2-[4-[2-(isothiocyanato-1,10-phenanthrolin-2-yl)ethynyl]anilino]-2-oxo-ethyl]amino]ethyl]amino]acetic acid **characterized by** formula 7.

2. A method according to claim 1, **characterized in that** in a step i) said 4-ethynylaniline is converted with a compound **characterized by** formula 10: 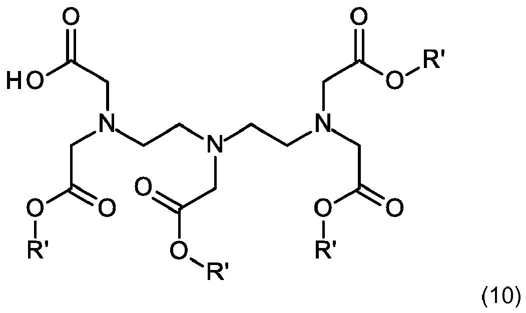 wherein R' has the same meaning as above,
to the educt of step g) said compound **characterized by** a formula 8.

3. A 2-[2-[bis(carboxymethyl)amino]ethyl-[2-[carboxymethyl-[2-[4-[2-(isothiocyanato-1,10-phenanthrolin-2-yl)ethynyl]anilino]-2-oxo-ethyl]amino]ethyl]amino]acetic acid **characterized by** formula 7.

4. A method for quantifying a biomolecule in a sample, comprising the steps:
- contacting a peptide with said biomolecule, wherein said peptide is labelled with a luminescent first FRET partner, and said peptide is bound by a specifically reactive ligand with a lower affinity than said biomolecule, and
- contacting said specifically reactive ligand with said peptide and said biomolecule, wherein said specifically reactive ligand is labelled with a second FRET-partner, and said first FRET partner and said second FRET partner are able to interact in such way that the luminescent signal of said first FRET partner is changed with spatial approximation of said first FRET partner and said second FRET partner, and
- quantifying the amount of the biomolecule by measurement of the luminescence of said first FRET partner,
**characterized in that**
said luminescent first FRET partner is a phosphorescent dye.

5. A method according to claim 4, wherein said phosphorescent dye is [EuL]H.

6. A method according to claim 4 or 5, wherein said peptide is obtained by conversion of the unlabelled peptide with 2-[2-[bis(carboxymethyl)amino]ethyl-[2-[carboxymethyl-[2-[4-[2-(isothiocyanato-1,10-phenanthrolin-2-yl)ethynyl]anilino]-2-oxoethyl]amino]ethyl]amino]acetic acid **characterized by** formula 7 or a compound **characterized by** a formula 12: 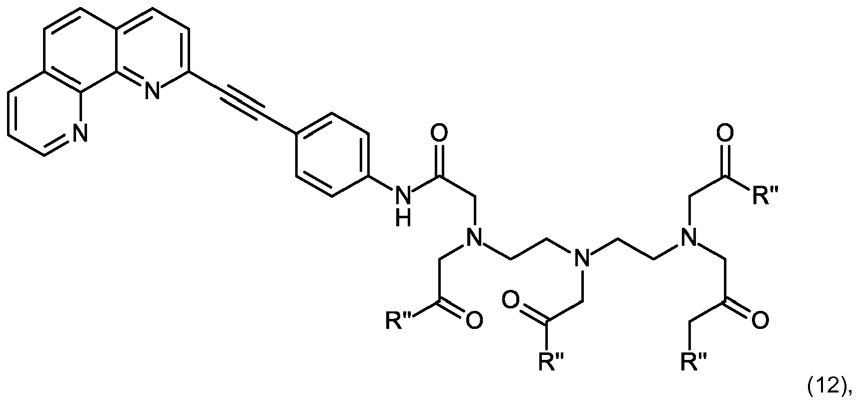 wherein each R" independently form another is selected from: 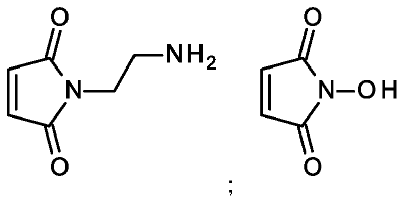 and OH.

7. A method according to any of claims 4 to 6, wherein said biomolecule is the tau protein.

8. A method according to any of claims 4 to 7, wherein said specifically reactive ligand is a monoclonal antibody.

9. A method according to claim 8, wherein said specifically ligand comprises or is a sequence selected from SEQ ID 1 and SEQ ID 2.

10. A method according to any of claims 4 to 9, wherein said peptide is VAVFR*pT*PPKSPSSAK-NH₂ or X-VAVFR*pT*PPKSPSSAK-NH₂, wherein X is mercaptopropionic acid and pT is phosphothreonine.

11. An ex *vivo* method for diagnosis of neurodegenerative diseases, comprising a method according to any of claims 4 to 10.

12. An ex *vivo* method according to claim 11, wherein said neurodegenerative disease is selected from the group comprised of Alzheimer's disease, Dementia pugilistica, frontotemporal dementia, parkinsonism linked to chromosome 17, Lytico-Bodig disease, tangle-predominant dementia, gangliocytoma, Meningioangiomatosis, subacute sclerosing panencephalitis lead encephalopathy, tuberous sclerosis, Hallervorden-Spatz disease, lipofuscinosis, Pick's disease and corticobasal degeneration.

13. A polypeptide, wherein said polypeptide comprises or is SEQ ID 1 or SEQ ID 2.


SEQUENCE LISTING

<110>  Universität Leipzig

<120>  Phosphorescent dye and phosphorescence immunoassay by peptide
       displacement

<130>  ule101ep

<160>  4

<170>  PatentIn version 3.5

<210>  1
<211>  120
<212>  PRT
<213>  Homo sapiens

<400>  1

Gln Val Thr Leu Lys Glu Ser Gly Pro Gly Ile Leu Gln Pro Ser Gln
1               5                   10                  15

Thr Leu Ser Leu Thr Cys Ser Phe Ser Gly Phe Ser Leu Ser Thr Ser
            20                  25                  30

Gly Met Gly Val Ser Trp Ile Arg Gln Pro Ser Gly Lys Gly Leu Glu
        35                  40                  45

Trp Leu Ala His Ile Tyr Trp Asn Asp Asp Lys Leu Tyr Asn Pro Ser
    50                  55                  60

Leu Lys Ser Arg Leu Thr Ile Ser Lys Asp Thr Ser Ser Asn Gln Val
65                  70                  75                  80

Phe Leu Lys Ile Thr Arg Val Asp Thr Ala Asp Thr Ala Thr Tyr Tyr
                85                  90                  95

Cys Val Arg Asp Gly Tyr Gly Tyr Asp Arg Phe Ala Tyr Trp Gly Gln
            100                 105                 110

Gly Thr Leu Val Thr Val Ser Ala
        115                 120


<210>  2
<211>  112
<212>  PRT
<213>  Homo sapiens

<400>  2

Asp Ile Val Met Thr Gln Ala Ala Pro Ser Val Pro Val Ile Pro Gly
1               5                   10                  15

Glu Ser Val Ser Ile Ser Cys Arg Ser Ser Lys Asn Leu Leu His Ser
            20                  25                  30

Asn Gly Asn Thr Tyr Val Tyr Trp Phe Leu Gln Arg Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Gly Ser Gly Ser Gly Thr Ala Phe Thr Leu Arg Ile
65                  70                  75                  80

Ser Arg Val Glu Ala Glu Asp Val Gly Ile Tyr Tyr Cys Met Gln His
                85                  90                  95

Leu Glu Tyr Pro Phe Thr Phe Gly Ser Gly Thr Lys Leu Glu Ile Lys
            100                 105                 110

<210>  3
<211>  360
<212>  DNA
<213>  Homo sapiens

<400>  3
caggttactc tgaaagagtc tggccctggg atattgcagc cctcccagac cctcagtctg       60

acttgttctt tctctgggtt ttcactgagc acttctggta tgggtgtgag ctggattcgt      120

cagccttcag gaaagggtct ggagtggctg gcacacattt attggaatga tgacaagctc      180

tataacccat ccctgaagag ccgactcaca atctccaagg atacctccag caaccaggta      240

ttcctcaaga tcaccagggt ggacactgca gatactgcca cttactactg tgttcgagat      300

ggctatggtt acgacaggtt tgcttactgg ggccaaggga ctctggtcac tgtctctgca      360

<210>  4
<211>  336
<212>  DNA
<213>  Homo sapiens

<400>  4
gatattgtga tgactcaggc tgcaccctct gtacctgtca ttcctggaga gtcagtatcc       60

atctcctgca ggtctagtaa gaatctccta catagtaatg gcaacactta tgtgtattgg      120

ttcctgcaga ggccaggcca gtctcctcag ctcctgatat atcggatgtc caaccttgcc      180

tcaggagtcc cagacaggtt cagtggcagt gggtcaggaa ctgctttcac actgagaatc      240

agtagagtgg aggctgaaga tgtgggaatt tattactgta tgcaacatct agaatatcca      300

ttcacgttcg gctcggggac aaagttggaa ataaaa                                336